# EUROPEAN PATENT APPLICATION

(11) **EP 3 928 748 A1**
(43) Date of publication of application: **29.12.2021**
(21) Application number: 20203652.1
(22) Date of filing: 23.10.2020
(51) Int. Cl.: A61F 2/58, B25J 9/00, B25J 9/10

(54) **EXOSKELETON GLOVE-TYPE ARTIFICIAL ARM**

(30) Priority: 26.06.2020 KR 20200078689
(71) Applicant: Mand.Ro Co., Ltd., Gyeonggi-do (KR)
(72) Inventor: YI, Sang Ho, Seoul (KR)
(74) Representative: Rückerl, Florian

(57) **Abstract**

The present invention relates to an artificial arm in which a finger is bent. The artificial arm includes an insertion portion (110) inserted into a tip of the finger, a tendon wire (w1) which is connected to a lower portion of the insertion portion and allows the insertion portion to perform a joint movement, an adjustment unit (180) which is located on a back of a hand and adjusts tension of the tendon wire, and a connection unit (160) which is made of an elastic material and having one end which is connected to the tendon wire so that an end of the tendon wire faces the adjustment unit and having the other end which is connected to the adjustment unit. According to the present invention, the finger is bent while the tendon wire is moved in an upward direction of the back of the hand along an edge portion of the hand, and in this process, a configuration that applies the tension to the tendon wire is located on the back of the hand, and thus an object is gripped more conveniently. In addition, in the present invention, the tendon wire is prevented from being loosened or from receiving excessive tension through the connection unit connected to the tendon wire, and thus a joint movement of the finger is induced more uniformly and durability is maximized.

## Description

### CROSS-REFERENCE TO RELATED APPLICATION

This application claims priority to and the benefit of Korean Patent Application No. 2020-0078689, filed on June 26, 2020, the disclosure of which is incorporated herein by reference in its entirety.

### BACKGROUND

### 1. Field of the Invention

The present invention relates to an exoskeleton glove-type artificial arm, and more particularly, to an artificial arm which assists in facilitating bending and spreading of fingers and in which power loss is minimized by controlling unnecessary driving thereof and durability is maximized.

### 2. Discussion of Related Art

Electronic prosthetics that replace amputated hands or legs of patients realize joint movements using motors or joint movements using strings or the like, depending on types thereof. In the case in which the joint movement is realized using the motors, various problems, such as requiring a considerably large amount of power because the motors are provided for each joint, burdening the wearer's physical strength because the electronic prosthesis itself is heavy, or the like, occur. In order to solve the above problems, a predetermined power value is pre-stored in the motor or the like to sense a limit value of the joint movement, and then the joint movement is further performed by the predetermined power value to realize a predetermined gripping force. However, in this case, there is a problem in that a sensor and a pre-stored predetermined power value should be provided and input in advance and it is difficult to realize various gripping forces according to situations or target objects.

In addition, there is a problem in that it is difficult for a patient whose hand is not amputated but who is unable to facilitate a joint movement of a finger due to damage to nerves, etc. to use a conventional artificial arm, and even when he or she uses the conventional artificial arm, the conventional artificial arm may act as an impeding factor that causes discomfort in movement due to its weight. Further, in the conventional artificial arm, a driving unit for driving the artificial arm is located in a palm portion, and thus there is a problem in that the patient can feel as though there's something foreign in his or her palm when gripping an object. Accordingly, there is a need for the development of artificial arms that is provided and used in a glove type so that fingers are inserted into a glove and joint movements of the fingers can be easily assisted and performed.

### [Document of Prior Art]

### [Patent Document]

Korean Registered Utility Model No. 20-0349089 (Registered on April 20, 2004)

### SUMMARY OF THE INVENTION

The present invention is directed to providing an artificial arm in which a joint movement and gripping force of a finger are realized uniformly.

The present invention is also directed to providing a technique in which a joint movement of a finger is easily induced regardless of the presence or absence of amputation of a hand.

The present invention is also directed to providing a technique in which feeling of discomfort due to occurrence of a sensation of there being something foreign in a palm portion while using an artificial arm is minimized.

According to an aspect of the present invention, there is provided an artificial arm, which is an artificial arm with bendable fingers. The artificial arm includes an insertion portion inserted into a tip of the finger, an adjustment unit provided at a position corresponding to a back of a hand and including a driving portion, a tendon wire having one end connected to a lower portion of the insertion portion and the other end wound around the driving portion, and a connection unit configured to guide the tendon wire moving in a downward direction of the finger from a predetermined reference position of a palm upward to an inside of the adjustment unit.

### BRIEF DESCRIPTION OF THE DRAWINGS

The above and other objects, features and advantages of the present invention will become more apparent to those of ordinary skill in the art by describing exemplary embodiments thereof in detail with reference to the accompanying drawings, in which:
FIG. 1 is a view entirely illustrating an appearance of wearing of an exoskeleton glove-type artificial arm according to an embodiment of the present invention;
FIG. 2 is a view entirely illustrating an exoskeleton glove-type artificial arm according to an embodiment of the present invention;
FIG. 3 is a view entirely illustrating an insertion portion and a restoration wire according to an embodiment of the present invention;
FIG. 4 is a view illustrating a tendon wire and a connection unit according to an embodiment of the present invention;
FIG. 5 is a view illustrating a tendon wire passing through a protection unit and a connection unit according to an embodiment of the present invention;
FIG. 6 is a view entirely illustrating an adjustment unit and a driving portion according to an embodiment of the present invention;
FIG. 7 is a view illustrating a driving portion according to an embodiment of the present invention; and
FIG. 8 illustrates cross-sectional views of an adjustment unit according to an embodiment of the present invention.

### DETAILED DESCRIPTION OF EXEMPLARY EMBODIMENTS

Hereinafter, embodiments of the present invention that are easily performed by those skilled in the art will be described in detail with reference to the accompanying drawings. However, the present invention may be implemented in several different forms and is not limited to the embodiments described herein or illustrated in the drawings. In addition, parts irrelevant to the present invention are omitted in the drawings in order to clearly explain the present invention. In the drawings, the same or similar reference numerals indicate the same or similar components.

Objects and effects of the present invention may be naturally understood or may become more apparent from the following description, and the objects and effects of the present invention are not limited only by the following description.

Before describing the present invention in detail, the term "upward direction" refers to a direction from a palm of a hand of a person toward a back of the hand and refers to a palm direction in which the palm faces the upward direction in a state in which the hand is turned upside down such that the palm faces the ground. In addition, the term "downward direction" refers to a direction opposite to the "upward direction" and refers to a direction in which a finger is bent so that a tip of the finger faces the ground.

Further, the term "joint movement" described in the present invention refers to a movement in which a finger is bent in a downward direction as an insertion portion and a cover unit are rotated around a joint of the finger.

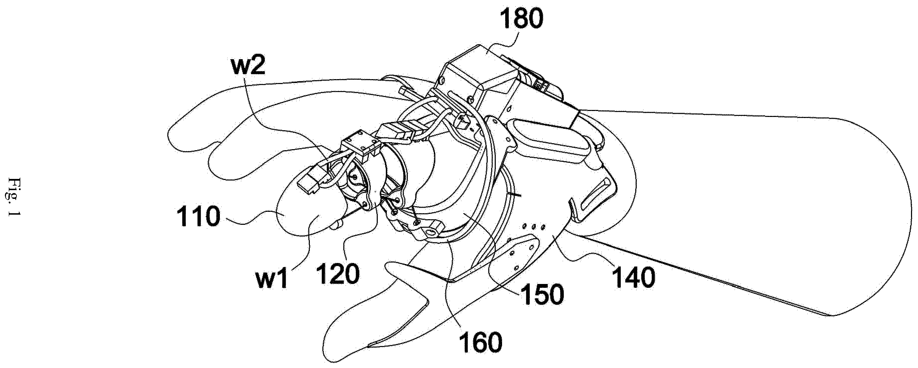

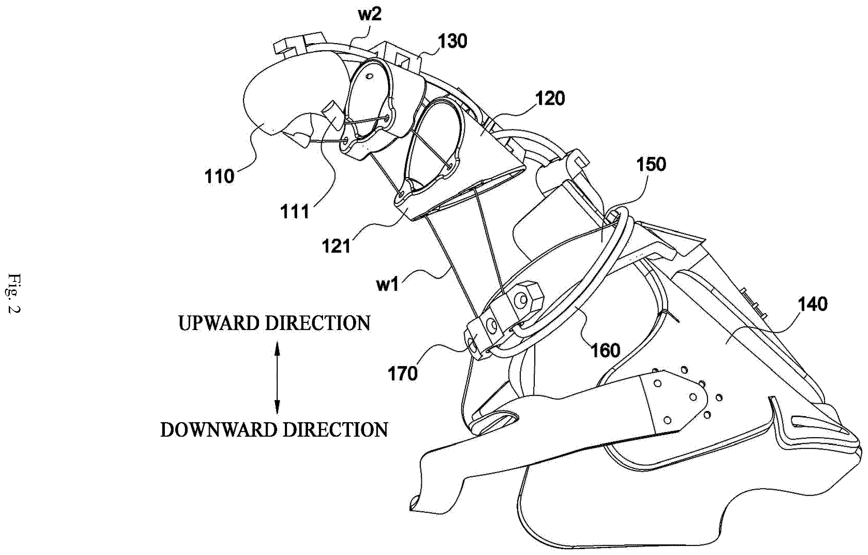

Hereinafter, the embodiments of the present invention will be described in detail with reference to the accompanying drawings. FIG. 1 is a view entirely illustrating an appearance of wearing of an exoskeleton glove-type artificial arm according to an embodiment of the present invention, and FIG. 2 is a view entirely illustrating the exoskeleton glove-type artificial arm according to the embodiment of the present invention.

The exoskeleton glove-type artificial arm according to the embodiment of the present invention is disclosed to bend a finger easily and includes an insertion portion 110 into which a tip of the finger is inserted, a tendon wire w1 which transmits a force to bend or spread the finger inserted into the insertion portion 110 to the insertion portion 110, an adjustment unit 180 which adjusts tension of the tendon wire w1, a connection unit 160 which connects the tendon wire w1 to the adjustment unit 180, and a support wire w2 which prevents sudden excessive bending of the finger.

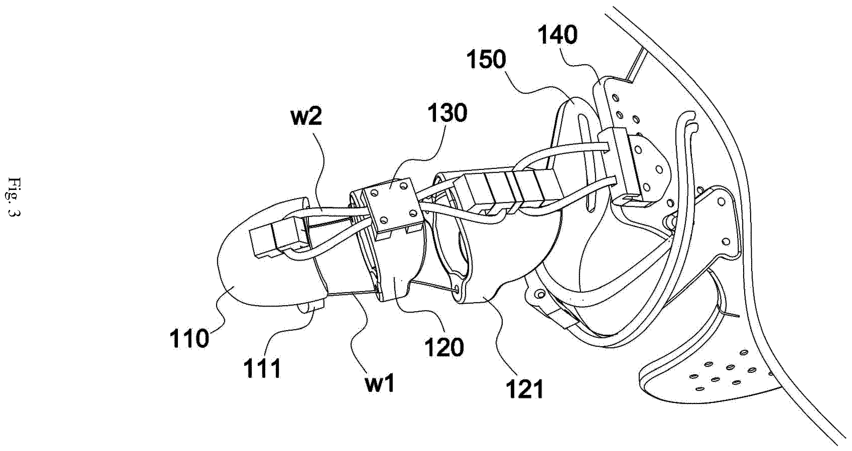

FIG. 3 is a view entirely illustrating the insertion portion 110 and a restoration wire according to the embodiment of the present invention.

The insertion portion 110 is a component for bending a finger. Specifically, the insertion portion 110 serves to spread or bend a knuckle where the tip portion of the finger is located through the tension due to the tendon wire w1 to be described below, in a state in which the tip of the finger is inserted therein. To this end, the insertion portion 110 is formed to have a thimble shape so as to correspond to the tip portion of the finger, a mounting portion 130 for mounting the support wire w2 to be described below is disposed in an upward direction of the insertion portion 110, and a first passage portion 111 for providing a path for the tendon wire w1 to stretch is provided in a downward direction of the insertion portion 110. By providing the insertion portion 110 as described above, a user who uses the artificial arm according to the present invention may easily transmit a force to a tip portion of his or her finger and bend the finger only by the tension of the tendon wire w1.

The first passage portion 111 protects the tendon wire w1 and provides a path for the tendon wire w1 to move or stretch in a process in which the tendon wire w1 connected to the insertion portion 110 is moved toward a lower side of the finger or passes through a back of the hand and is moved toward an upper side of a body portion 140 and is connected to a driving portion 181. That is, the first passage portion 111 prevents the tendon wire w1 from being unintentionally moved in the upward direction of the finger or from being caught and damaged by an external object in the process in which the tendon wire w1 is moved in a downward direction of the finger. To this end, the first passage portion 111 is located in the downward direction of the insertion portion 110, and the tendon wire w1 is disposed so as to pass in a direction in which a second passage portion 121 is located in a state in which one end of the tendon wire w1 is connected to the first passage portion 111. Accordingly, the tendon wire w1 is connected to the first passage portion 111 which is attached and formed in the downward direction of the insertion portion 110. As a further exemplary embodiment of the present invention, the first passage portion 111 may be provided in a pair for easier tension action of the tendon wire w1 and is disposed on each of both side surfaces of the insertion portion 110. In the case of the present invention including the first passage portion 111 as described above, the insertion portion 110 may be bent easily even with a smaller force as compared to a case in which the tendon wire w1 is provided to surround the insertion portion 110. Further, in the present invention, since the tendon wire w1 is provided so as not to surround the insertion portion 110, it is possible to prevent the tendon wire w1 from being damaged when an object is caught on the tip of the finger.

The cover unit 120 provides a path for the tendon wire w1 to stretch in the process in which the finger inserted into the insertion portion 110 is bent or spread by the tendon wire w1. Specifically, the cover unit 120 prevents the tendon wire w1, which may be loosened due to a decrease in tension caused by the joint movement of the finger, from being caught by an external object or from being moved to an unintended position. To this end, the cover unit 120 is provided to be fitted onto the finger and the second passage portion 121 is provided in a downward direction of the cover unit 120, and thus the cover unit 120 is connected to the tendon wire w1. Specifically, the cover unit 120 is provided by being fitted onto each knuckle of the finger so as to correspond to each knuckle of the finger. Accordingly, the tendon wire w1 is formed to extend from the insertion portion 110 toward a coupling unit 150 and the body portion 140 and is inserted into the connection unit 160 to be described below by sequentially passing through the above-described first and second passage portions 111 and 121. By providing the cover unit 120 as described above, the tendon wire w1 is prevented from being excessively loosened by an external object or from being separated from the artificial arm in the process in which the user bends or spreads the finger, and thus durability may be significantly improved. Further, it is possible to provide a more secure path for the tendon wire w1 to stretch through the cover unit 120 and the second passage portion 121, and it is possible to provide a more accurate tension to the tendon wire w1. Meanwhile, the mounting portion 130 for preventing the separation of the support wire w2 is further provided in the upward direction of the cover unit 120.

The mounting portion 130 prevents the support wire w2 from being separated in the process in which the support wire w2 serves to prevent the insertion portion 110 and the cover unit 120 from being excessively bent. Specifically, the mounting portion 130 provides a stretched position of the support wire w2 and prevents the support wire w2 from being separated from the insertion portion 110 and the cover unit 120. To this end, the mounting portion 130 is provided in each of the insertion portion 110 and the cover unit 120 and is located in the upward direction of each of the insertion portion 110 and the cover unit 120. Specifically, the mounting portions 130 are divided into a first mounting portion 130 located in the upward direction of the insertion portion 110 and a second mounting portion 130 located in the upward direction of the cover unit 120. Further, separate opening grooves through which the support wire w2 passes are provided in both side directions of the first mounting portion 130 and in front (a left direction of FIG. 3) and rear (a right direction of FIG. 3) surfaces of the second mounting portion 130. Accordingly, the support wire w2 passes through the opening groove provided in the first mounting portion 130 and the opening groove provided in the second mounting portion 130 sequentially and is connected to a separate fixing unit provided in the body portion 140, and thus end portions thereof are fixed.

The tendon wire w1 is a component for inducing the joint movement of the finger inserted into the insertion portion 110 and the cover unit 120 as described above and is connected to the insertion portion 110, the passage portion, and the cover unit 120 sequentially. Further, the tendon wire w1 is provided as one wire, wherein one end of the wire is connected to a lower portion of the insertion portion 110, and the other end is connected to the driving portion 181 and is provided to be wound around the driving portion 181. Therefore, an effect, in which the accuracy of the joint movement of the finger that occurs as the tension is provided by using one tendon wire w1 is significantly increased, may be obtained.

The support wire w2 serves to prevent the finger from excessively bending and to compensate for a force when the finger is spread in the process of the joint movement of the finger. To this end, the support wire w2 is connected to a separate fixing unit located at the rear by sequentially passing through the opening grooves provided in the first mounting portion 130 and the second mounting portion 130 described above. Further, the support wire w2 is made of a material having a restoring force, such as rubber or the like. Accordingly, both end portions of the support wire w2 may prevent the finger from being excessively bent while being connected to the fixing unit and may bring the insertion portion 110 and the cover unit 120, which are in a bent state, back to their original positions using the restoring force so as to be located on a straight line. Accordingly, an effect in which power required for the joint movement of the finger is minimized may be obtained.

The body portion 140 provides a space in which the insertion portion 110 and the cover unit 120 integrally perform a joint movement. Specifically, the body portion 140 is a glove portion of the exoskeleton glove-type artificial arm according to the present invention and is provided to surround the back of the hand, and the cover unit 120 and the insertion portion 110 are located in front (which refers to the left direction of FIG. 3) thereof. Further, the adjustment unit 180 including the driving portion 181 to be described below is provided on the back of the hand of the body portion 140. Specifically, the driving portion 181 which adjusts the tension by winding the tendon wire w1 is located in an upward direction of the body portion 140. Accordingly, the adjustment unit 180 may be fixedly located on the back of the hand. That is, in the artificial arm according to the present invention, it is possible to use the artificial arm more conveniently because the driving unit is not mounted on the palm portion. A more detailed description thereof will be given below.

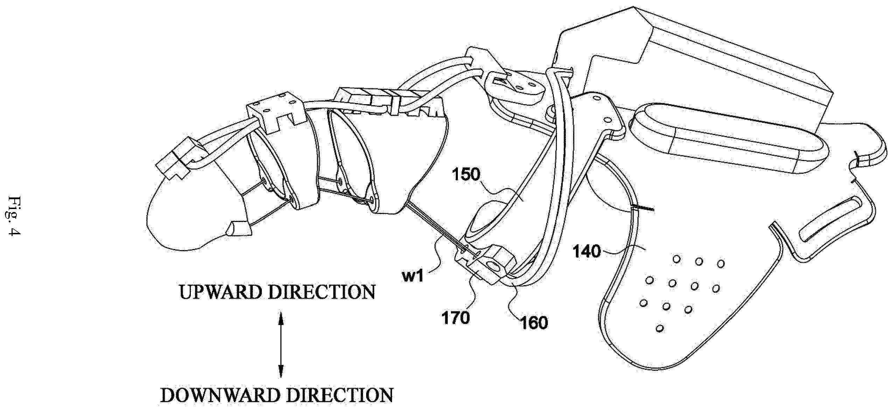

The coupling unit 150 is a component which fixes the body portion 140 to the back of the hand. Specifically, the coupling unit 150 is formed to extend from the body portion 140 and is provided such that the back and the palm of the hand are inserted and located between the coupling unit 150 and the body portion 140. Specifically, the coupling unit 150 is formed to have a U shape, an L shape, or the like so as to be curved and to extend from the body portion 140 to a predetermined reference position L, and one surface thereof facing the upward direction of the coupling unit 150 is brought into contact with the palm. Accordingly, the coupling unit 150 may allow the protection unit 170, which will be described below in FIG. 4, to be located in the downward direction thereof, and allow the tendon wire w1 to pass through the protection unit 170 by sequentially passing through the first passage portion 111 and the second passage portion 121 to correspond to the coupling unit 150. Specifically, the tendon wire w1 passes through the protection unit 170 disposed at the predetermined reference position L, is then guided upward toward the back of the hand, and thus is wound around the driving portion 181.

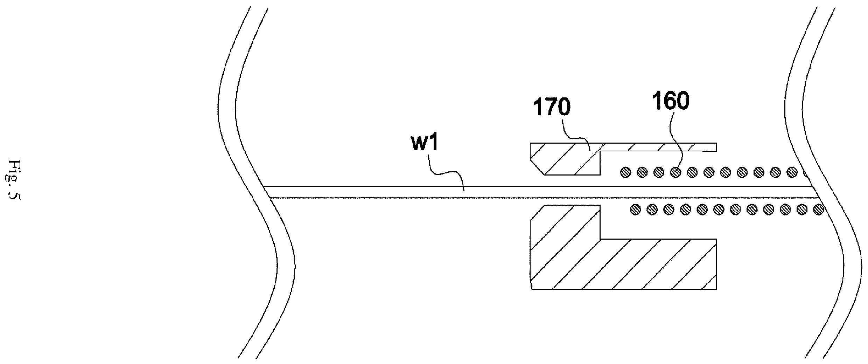

FIG. 4 is a view illustrating the tendon wire w1 and the connection unit 160 according to the embodiment of the present invention, and FIG. 5 is a view illustrating the tendon wire w1 passing through the protection unit 170 and the connection unit 160 according to the embodiment of the present invention.

The connection unit 160 is a component which adjusts the tension of the tendon wire w1. Specifically, as the tendon wire w1 passing through an inside of the connection unit 160 is wound or unwound by the driving portion 181, the connection unit 160 controls the tension of the tendon wire w1 that is integrally moved. More specifically, the connection unit 160 guides the tendon wire w1, which is moved in the downward direction of the finger, from the predetermined reference position L of the palm upward to the inside of the adjustment unit 180. Further, the connection unit 160 performs a protective function in which the tendon wire w1, which is guided upward from the downward direction of the palm to the back of the hand, is prevented from being caught by an external object or from being damaged by an external force. To this end, the connection unit 160 is provided as a pair of connection units and is integrally provided with the tendon wire w1 as one end is connected to the tip of the tendon wire w1. Here, the term "reference position L" refers to a point at which the protection unit 170 located in the downward direction of the above-described coupling unit 150 is disposed. More accurately, the term "reference position L" is formed to be curved and extend from the body portion 140 on the back of the hand, is provided as a midpoint of the coupling unit 150 located in the palm direction by a predetermined interval from a first knuckle of the finger where the finger and the palm are connected to each other, and thus the protection unit 170 may be located at the reference position L. However, the reference position L as described above is not limited to the midpoint of the coupling unit 150 and may be provided as a position offset from the midpoint of the coupling unit 150 toward a direction of an edge or thumb of the hand according to the position of the finger. Further, the connection unit 160 is provided with a spring or the like that can be stretched and has an open inside, and thus the tendon wire w1, which passes through the first passage portion 111 located in the downward direction of the insertion portion 110 and the second passage portion 121 located in the downward direction of the cover unit 120 sequentially, is guided upward while being inserted into the connection unit 160 at the reference position L and is wound around the driving portion 181. Further, the connection unit 160 is provided to have an elastic material that can be stretched. Further, the other end of the connection unit 160 is connected to the adjustment unit 180. Specifically, when the tendon wire w1 is wound in response to the rotational movement of a roller in a state in which the other end of the connection unit 160 is connected to the adjustment unit 180, the connection unit 160 is reduced to further pull the tendon wire w1 to the rear where the wrist is located. Corresponding to the above configuration, the insertion portion 110 and the cover unit 120 which are connected to the tendon wire w1 perform a joint movement in which the finger is bent toward the palm. By providing the connection unit 160 as described above, the tendon wire w1 may be protected from the external force. Further, when compared to the case of performing the joint movement of the finger only with the tendon wire w1 without the connection unit 160, a sagging phenomenon that may occur when a length of the tendon wire w1 is increased may be prevented so that a joint movement may be performed on the insertion portion 110 and the cover unit 120 by only applying a small power.

The protection unit 170 is a component which protects a portion in which the tendon wire w1 and the connection unit 160 are connected to each other. Specifically, the protection unit 170 protects the tendon wire w1, which is inserted into the connection unit 160, and one end of the protection unit 170 from an external force and prevents the connection unit 160 from being excessively stretched by fixing a position of one end of the connection unit 160. To this end, the protection unit 170 is located in the downward direction of the coupling unit 150 described above and has a through hole. Further, the protection unit 170 is located at the above-described reference position L and is located in the downward direction of the coupling unit 150. That is, the protection unit 170 is formed integrally with the coupling unit 150 and is disposed at a point corresponding to the reference position L. Accordingly, one end of the connection unit 160 is inserted into and fixed to the protection unit 170, and at the same time, the tendon wire w1 disposed to face the reference position L passes through the through hole, is introduced into the connection unit 160, is raised toward the back of the hand, and is moved toward the driving portion 181. Therefore, it is possible to prevent the tendon wire w1 and the connection unit 160 from being disconnected and separated from each other by an external force.

According to the embodiment of the present invention, the number of each of the driving portion 181, the tendon wire w1, and the connection unit 160 described above is one. Specifically, in the embodiment of the present invention, one tendon wire w1, one connection unit 160 into which the one tendon wire w1 is inserted, and one driving portion 181 which winds the one tendon wire w1 may be provided. In this case, the tendon wire w1 passes through one first passage portion 111 provided in the downward direction of the insertion portion 110, one second passage portion 121 provided in the downward direction of the body portion 140, and the protection unit 170 formed in the downward direction of the body portion 140 so as to correspond to the reference position L. Further, the one tendon wire w1 passing through the protection unit 170 is guided upward from the palm toward the back of the hand while being inserted into the one connection unit 160 and is wound around the one driving portion 181. That is, the tendon wire w1 has a structure in which one end of the tendon wire w1 is connected to the first passage portion 111 located in the downward direction of the insertion portion 110 and the other end is wound around the driving portion 181. According to the embodiment of the present invention as described above, only a minimum configuration for the joint movement of the finger may be disposed, thereby simplifying the artificial arm.

As still another embodiment of the present invention, the tendon wire w1, the first passage portion 111, the second passage portion 121, and the connection unit 160 may each be provided in a pair. As yet another embodiment of the present invention, one tendon wire w1, one driving portion 181, and one connection unit 160 are formed as one winding group, and the winding group is provided with a plurality of winding groups and is formed as a first group and a second group. In this case, one end of a first tendon wire w1 belonging to the first group and one end of a second tendon wire w1 belonging to the second group are connected to a pair of first passage portions 111 located in the downward direction of the insertion portion 110, and thus each of the one ends may be fixed. That is, the one end of the first tendon wire w1 and the one end of the second tendon wire w1 are connected to the first passage portions 111 in one-to-one correspondence therewith. Thereafter, the first tendon wire w1 and the second tendon wire w1 pass through a pair of second passage portions 121 and pass through a pair of through holes provided in the protection unit 170. Further, the first tendon wire w1 and the second tendon wire w1 are disposed at reference positions L, are inserted into a first connection unit 160 belonging to the first group and a second connection unit 160 belonging to the second group, respectively, and are guided upward. Thereafter, the other end of the first tendon wire w1 and the other end of the second tendon wire w1, which are introduced into the adjustment unit 180, are wound around a first driving portion 181 belonging to the first group and a second driving portion 181 belonging to the second group, respectively, and thus the joint movement of the finger starts. Through the first tendon wire w1 and the second tendon wire w1 as described above, the insertion portion 110 and the cover unit 120 may allow the joint movement to be easily performed with a smaller force, thereby maximizing efficiency. Further, since the two tendon wires w1 are provided, the joint movement of the finger may be realized with the other tendon wire w1 even when a problem occurs in one tendon wire w1, and thus durability may be significantly improved.

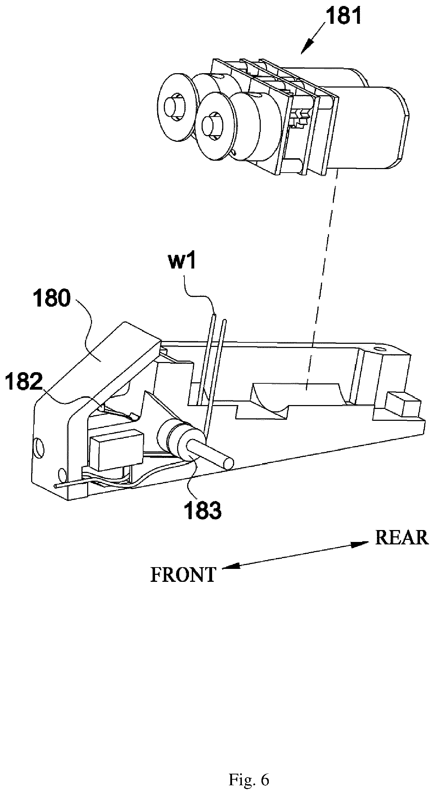

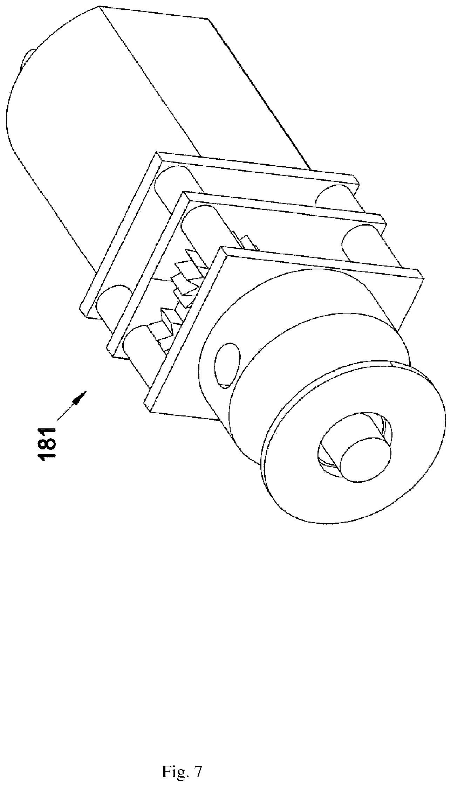

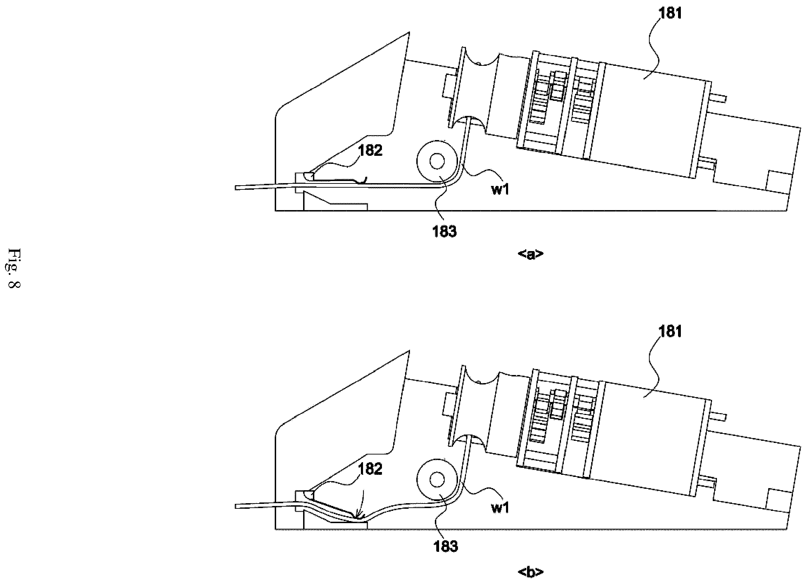

FIG. 6 is a view entirely illustrating the adjustment unit 180 and the driving portion 181 according to the embodiment of the present invention, FIG. 7 is a view illustrating the driving portion 181 according to the embodiment of the present invention, and FIG. 8 illustrates cross-sectional views of the adjustment unit 180 according to the embodiment of the present invention.

The adjustment unit 180 provides a space in which the joint movement of the finger is realized by adjusting the tension of the tendon wire w1. The adjustment unit 180 is located in the upward direction of the body portion 140 and disposed on the back of the hand and adjusts the tension of the tendon wire w1 using the driving portion 181 provided as a small motor. To this end, the adjustment unit 180 includes a driving portion 181, a sensing portion 182, and a tension holding portion 183.

The driving portion 181 is a component, which is provided as a power generating unit such as a small motor or the like and rotates a roller separately provided through a gear by driving. To this end, the driving portion 181 is disposed so as to be accommodated in the adjustment unit 180. Further, the roller provided in the driving portion 181 passes through the connection unit 160 and winds and unwinds the tendon wire w1 guided upward through rotation. To this end, the other end of the tendon wire w1 is connected to the roller, is rotated using the power generated from the driving portion 181, and adjusts the tension by winding and unwinding the tendon wire w1.

The sensing portion 182 is provided to sense that the tension of the tendon wire w1 introduced into the adjustment unit 180 is small and the tendon wire w1 sags in the downward direction. Specifically, the sensing portion 182 senses release of contact with the tendon wire w1 having a small tension. To this end, the sensing portion 182 is located inside the adjustment unit 180 and is located in front (which refers to a left direction of FIG. 8) of the roller. Further, the sensing portion 182 is located in the upward direction of the tendon wire w1 entering the inside of the adjustment unit 180 so that one end thereof is in contact with the tendon wire w1. Here, the sensing portion 182 senses the loosening of the tendon wire w1 through interaction with a separate sensing unit which is located inside the adjustment unit 180 to be located in the downward direction.

FIG. 8A illustrates a case in which the tendon wire w1 enters the inside of the adjustment unit 180 while maintaining a tense state, wherein the sensing portion 182 maintains a state not in contact with the separate sensing unit. On the other hand, FIG. 8B illustrates a case in which the tendon wire w1 is loosened and sags in the downward direction and the sensing portion 182 presses against the tendon wire w1 in the downward direction. In this case, when the sensing portion 182 approaches the separate sensing unit disposed in the downward direction, the sensing portion 182 may sense that the tension of the tendon wire w1 is reduced.

By providing the sensing portion 182, it is possible to easily prevent the tendon wire w1 from being loosened and to prevent occurrence of a situation in which the tendon wire w1 is caught and disconnected from an external object or the like. Further, even when the joint movement of the finger is not performed, the tension of the tendon wire w1 may be uniformly maintained.

The tension holding portion 183 maintains the tension of the tendon wire w1 constant. Further, the tension holding portion 183 is provided to block a situation in which the tendon wire w1 is released inside the adjustment unit 180 or is pulled into a gear in the driving portion 181 to cause a failure. To this end, the tension holding portion 183 is located between the roller and the sensing portion 182 (refer to FIG. 8). Further, the tension holding portion 183 is formed to have a cylindrical shape and is provided such that an outer circumferential surface thereof is in surface contact with the tendon wire w1 located in the downward direction. Accordingly, the tendon wire w1 in contact with the tension holding portion 183 may be wound and unwound at a uniform speed to correspond to the driving of the driving portion 181 and the rotation of the roller, and convenience in the joint movement process of the finger may be maximized. In addition, when compared to a case in which the tension holding portion 183 is not provided, it is possible to prevent the tension of the tendon wire w1 from indiscriminately changing and at the same time, it is possible to induce the tendon wire w1 to be wound and unwound, thereby enabling the joint movement of the finger more easily.

According to the present invention, a finger is bent while a tendon wire is moved in an upward direction of a back of a hand along an edge portion of the hand, and in this process, a configuration that applies tension to the tendon wire is located on the back of the hand, and thus an object can be gripped more conveniently.

Further, in the present invention, it is possible to prevent a tendon wire from being loosened or from receiving excessive tension through a connection unit connected to the tendon wire and at the same time, it is possible to protect the tendon wire, and thus a joint movement of a finger can be induced more uniformly and durability can be maximized.

While the present invention has been described with reference to the exemplary embodiments, these are only exemplary. It will be understood by those skilled in the art that various modifications and equivalent other examples may be made. Therefore, the scope of the present invention is not limited by the above-described embodiments and the accompanying drawings.

## Claims

1. An exoskeleton glove-type artificial arm comprising:
an insertion portion (110) inserted into a tip of a finger;
an adjustment unit (180) provided at a position corresponding to a back of a hand and including a driving portion (181);
a body portion (140) provided to surround the back of the hand and configured to fix the position of the adjustment unit (180);
a coupling unit (150) formed to be curved so as to extend from the body portion (140) to a predetermined reference position (L); and
a tendon wire (w1) having one end connected to a lower portion of the insertion portion (110) and the other end connected to the driving portion (181),
wherein the tendon wire (w1) is connected to the coupling unit (150) and guided upward along the body portion (140) to be wound around the driving portion (181).

2. The exoskeleton glove-type artificial arm of claim 1, further comprising a connection unit (160) into which the tendon wire (w1) moving in a downward direction of the finger is inserted and which guides the tendon wire (w1) upward to an inside of the adjustment unit (180) at the reference position (L),
wherein the connection unit (160) has one end located in a downward direction of the coupling unit (150) corresponding to the reference position (L).

3. The exoskeleton glove-type artificial arm of claim 2, further comprising a protection unit (170) located in the downward direction of the coupling unit (150) and connected to the one end of the connection unit (160).

4. The exoskeleton glove-type artificial arm of claim 2, further comprising a cover unit (120) provided by being fitted onto the finger so as to correspond to each knuckle of the finger and connected to the tendon wire (w1).

5. The exoskeleton glove-type artificial arm of claim 4, further comprising:
a first passage portion (111) located in the downward direction of the insertion portion (110); and
a second passage portion (121) located in a downward direction of the cover unit (120),
wherein the tendon wire (w1) is inserted into the connection unit (160) by passing through the first passage portion (111) and the second passage portion (121) sequentially in a state in which one end thereof is connected to the first passage portion (111).

6. The exoskeleton glove-type artificial arm of claim 1, wherein the number of each of the driving portion (181), the tendon wire (w1), and a connection unit (160) is one.

7. The exoskeleton glove-type artificial arm of claim 1, wherein:
a winding group including the driving portion (181), the tendon wire (w1), and a connection unit (160) is divided into a first group and a second group; and
one end of the tendon wire (w1) in each of the first group and the second group is fixed in the downward direction of the insertion portion (110).
